# EUROPÄISCHE PATENTANMELDUNG

(11) **EP 0 829 225 A2**
(43) Veröffentlichungstag der Anmeldung: **18.03.1998**
(21) Anmeldenummer: 97114115.5
(22) Anmeldetag: 14.08.1997
(51) Int. Cl.: A61B 5/00

(54) **Messkopf für die photoakustische Spektroskopie**

(30) Priorität: 14.08.1996 DE 19632867
(71) Anmelder: Columbus Schleif-und Zerspantechnik Hard-und Software GmbH, 87634 Obergünzburg (DE)
(72) Erfinder: Zürl, K. Dr.Ing., 87435 Kempten (DE); Weiss, Armin, 82467 Garmisch Partenkirchen (DE); Zobel, F., 87496 Untrasried (DE); Boss, Karl-Siegfried Prof., 82131 Gauting (DE)
(74) Vertreter: VOSSIUS & PARTNER

(57) **Zusammenfassung**

Es wird ein Meßkopf für die photoakustische Spektroskopie zur Verfügung gestellt, wobei mindestens eine Strahlungsquelle in dem Meßkopf angeordnet ist. Die Vorteile der Erfindung liegen in der preisgünstigen Herstellung des Meßkopfs und einer einfachen Handhabung.

## Beschreibung

Die Erfindung betrifft einen Meßkopf für die photoakustische Spektroskopie (PAS). Ein solcher Meßkopf kann insbesondere bei der zerstörungsfreien und kontaminationsfreien Untersuchung von menschlichem Blut oder Gewebe in der Medizin zum Einsatz kommen.

Die PAS ist eine bekannte spektroskopische Untersuchungsmethode, für Gase Flüssigkeiten und Festkörper. Spektral schmalbandige Strahlung wird in ein Meßobjekt eingestrahlt und zum Teil absorbiert, d.h. in Wärme umgesetzt. Ein Teil dieser Wärme tritt an der Oberfläche des Objekts aus und die Energie wird an die umgebende Luft (Mittlergas) abgegeben. Wird die Strahlungsquelle periodisch in ihrer Intensität moduliert, so erfolgt die Energieabgabe periodisch, und es entsteht eine Schallwelle mit der Frequenz der Modulation der Strahlungsquelle. Diese Schallwelle wird durch eine Meßkammer auf einen Schallaufnehmer geleitet und ausgewertet. In der Regel ist der Schalldruck bzw. die Ausgangsspannung des Schallaufnehmers die interessierende Kenngröße, die mit den spektralen Absorptionseigenschaften des zu untersuchenden Materials zusammenhängt. Die Messung wird im allgemeinen mit schmalbandiger Strahlung verschiedener Wellenlängen wiederholt.

Aus der DE-C2-44 00 674 ist ein photoakustischer Sensor bekannt. Dieser weist ein Element, das eine elektromagnetische Strahlung emittiert, und ein druckempfindliches Element zum Nachweis eines in einer Probe angeregten photoakustischen Signals auf. Es ist jedoch nur eine Strahlungsquelle außerhalb des Meßkopfes vorgesehen, die über ein Linsensystem Strahlung in einem tiefen Bereich der Probe fokussiert. Die Strahlung wird über eine Glasfaser an den Meßkopf herangeführt.

Demgegenüber liegt der Erfindung die Aufgabe zugrunde, einen Meßkopf für die photoakustische Spektroskopie zur Verfügung zu stellen, der einfach aufgebaut ist, preisgünstig ist, und eine einfache Handhabung erlaubt.

Die Aufgabe wird mit den Merkmalen der Patentansprüche gelöst.

Bei der Lösung geht die Erfindung von dem Grundgedanken aus, die Strahlungsquelle, bevorzugt mehrere Strahlungsquellen im Meßkopf selbst anzuordnen.

Die Vorteile der Erfindung sind:
- Günstige und kompakte Montage auch für kleine auch beweglich einsetzbare Meßköpfe;
- Preisgünstige Herstellung, da auf Mittel zur Strahlungsführung, wie Glasfasern, verzichtet werden kann;
- auf strahlformende optische Elemente kann verzichtet werden, oder diese Elemente bilden gleichzeitig die Meßzelle;
- die Montageebene der Strahlungsquellen kann Teil der Meßzelle sein; und
- bei der Verwendung von LED's oder Laserdioden als Strahlungsquelle ergibt sich eine preisgünstige Montage der Komponenten (chip-on-board-Technologie) bei sehr kleiner möglicher Bauform des Meßkopfes.

Im folgenden wird die Erfindung anhand der Zeichnungen näher erläutert. Es zeigen:
- Fig. 1: eine erste erfindungsgemäße Ausführungsform,
- Fig. 2: eine zweite erfindungsgemäße Ausführungsform,
- Fig. 3: eine dritte erfindungsgemäße Ausführungsform,
- Fig. 4: eine vierte erfindungsgemäße Ausführungsform, und
- Fig. 5: eine erfindungsgemäße Ausführungsform einer Photonenfalle vor dem Schallaufnehmer.

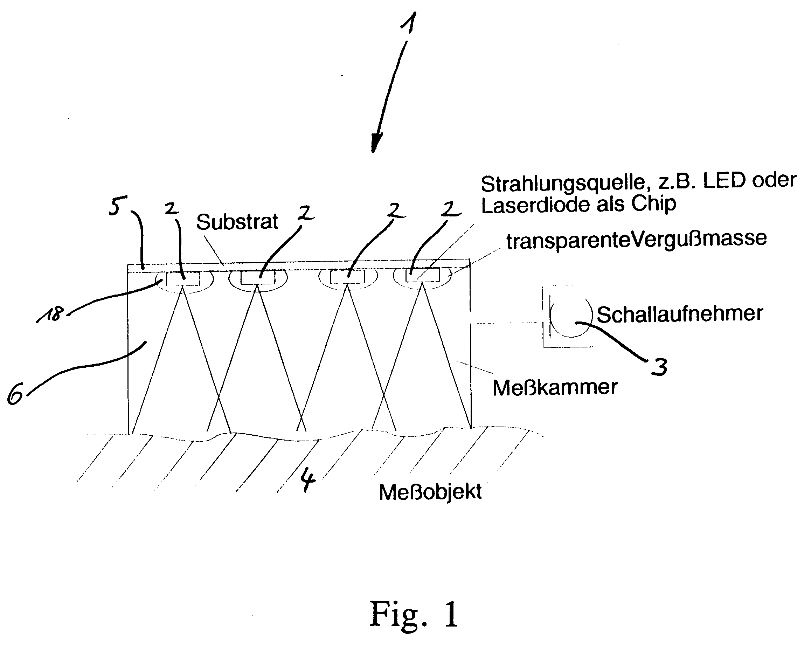

Fig. 1 zeigt eine erste erfindungsgemäße Ausführungsform, bei der mehrere Strahlungsquellen 2 als Chips direkt auf einem Substrat 5 angeordnet sind, welches auch als Abschluß der Meßkammer dient. Zum mechanischen Schutz und zur besseren Wärmeabfuhr sind die Chips mit einer transparenten Vergußmasse 18 bedeckt. Wenn es sich bei den Strahlungsquellen 2 um Laserdioden handelt, so ist ein Umlenkspiegel erforderlich, um die Strahlung auf das Objekt 4 zu lenken. Es können weiterhin LED's zum Einsatz kommen oder auch oberflächenemittierende Laserdioden, sogenannte VCSEL (Vertical Cavity Surface Emitting Laserdiodes) verwendet werden. Die durch die Strahlung induzierten Schallwellen vom Meßobjekt 4 werden zu dem Schallaufnehmer 3 geleitet und dann zur Auswertung der in ihnen enthaltenen Informationen vom Meßobjekt weitergeleitet. Der Meßkopf gemäß der ersten erfindungsgemäßen Ausführungsform ist besonders preisgünstig durch die Anordnung der Strahlungsquellen als Chips direkt auf einem Substrat und kann somit in Massenproduktion hergestellt werden.

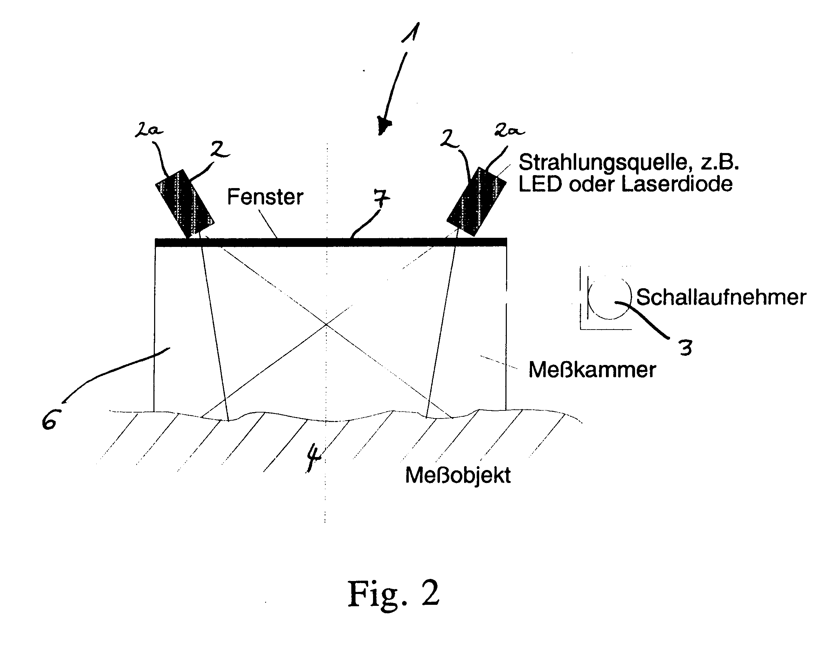

Fig. 2 zeigt eine zweite erfindungsgemäße Ausführungsform. In dieser Ausführungsform sind mehrere Strahlungsquellen 2 in Gehäusen 2a rotationssymmetrisch oberhalb der Meßkammer 6 angeordnet. Eine optisch transparente Platte 7 (Glas oder ähnliches) bilden den oberen Abschluß der Meßkammer 6 und verhindert eine mechanische Beschädigung der Strahlungsquellen 2 z.B. bei der Reinigung der Meßkammer.

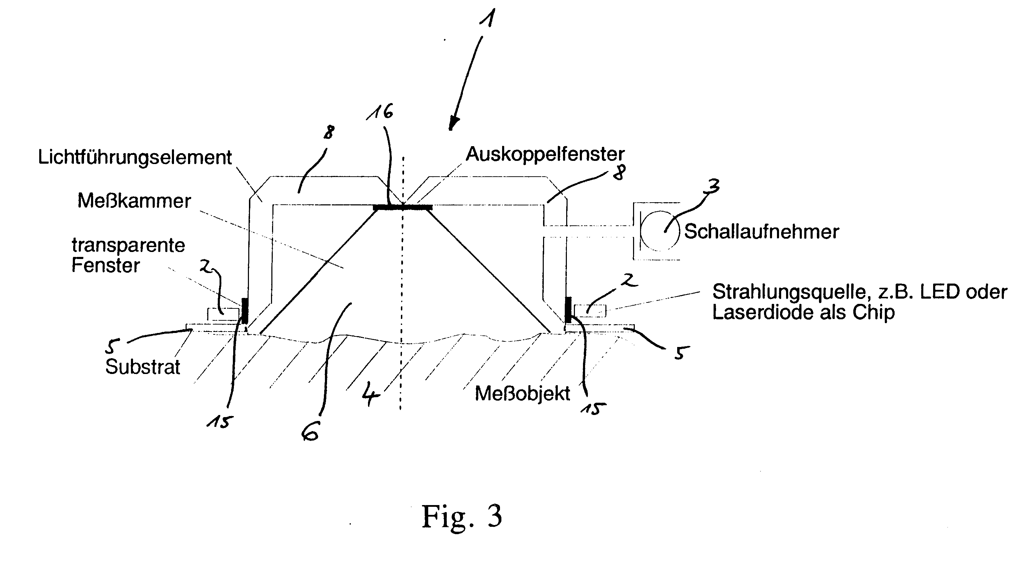

In Fig. 3 ist eine dritte erfindungsgemäße Ausführungsform dargestellt. Die Meßkammer 6 ist von einem Lichtführungselement 8 umgeben. Die Strahlungsquellen 2 befinden sich außerhalb der Meßkammer 6 auf einem Substrat 5 und in optischer Verbindung mit dem Lichtführungselement 8 durch transparente Fenster 15. Das Lichtführungselement 8 kann aus transparentem Kunststoff bestehen. Das Licht wird in dem Element durch Totalreflexion geführt, die durch einen geeigneten Brechungsindex des Materials oder durch eine Verspiegelung z.B. durch Verdampfen erreicht wird. Das Licht im Lichtführungselement 8 koppelt die Strahlung im Fenster 16 aus und sendet sie auf das Meßobjekt 4. Der Vorteil dieser Ausführungsform besteht in der direkten Verwendung des Lichtführungselements 8 als Wandung der Meßkammer 6. Außerdem können alle Strahlungsquellen 2 in einer Ebene als Chips auf dem Substrat 5 aufgebracht werden. Es können dabei konventionelle, kantenemittierende Laserdioden-Chips eingesetzt werden, da die Funktion eines Umlenkspiegels im Lichtführungselement 8 integriert sein kann.

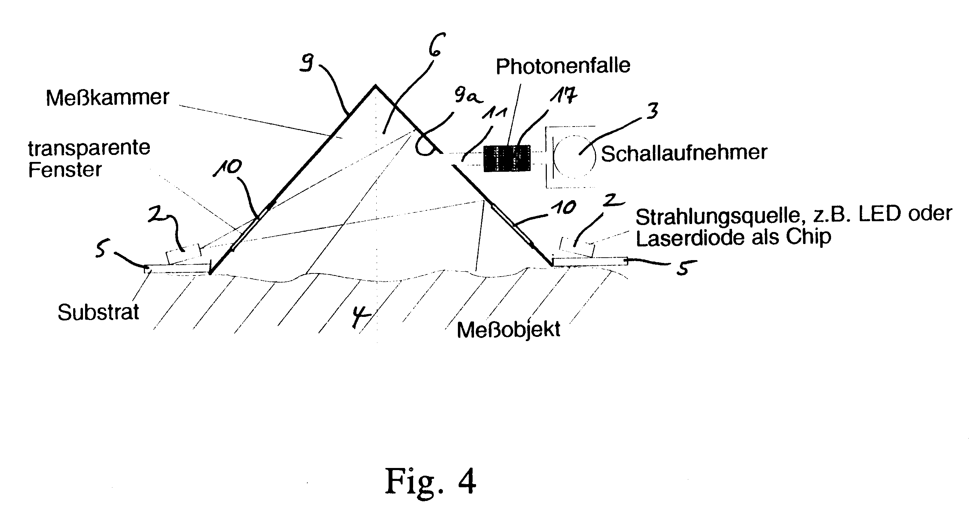

Fig. 4 zeigt eine vierte erfindungsgemäße Ausführungsform. Die Meßkammer 6 besteht aus einem pyramidenförmigen Hohlkörper 9. Mehrere Strahlungsquelle 2 sind auf einem Substrat angeordnet, das den Hohlkörper 9 umgibt. Die Strahlung jeder Strahlungsquelle 3 dringt durch ein Fenster 10 in den Hohlkörper 9, der an der Innenseite 9a verspiegelt ist, ein und wird von dort auf das Meßobjekt 4 reflektiert. Dabei ergibt sich für alle Strahlungsquellen 2 ein im wesentlichen identischer Bestrahlungsfleck auf dem Meßobjekt 4. Eine Photonenfalle 17 verhindert einen ungewünschten photoakustischen Effekt im Kanal 11 zwischen der Meßkammer 6 und dem Schallaufnehmer 3.

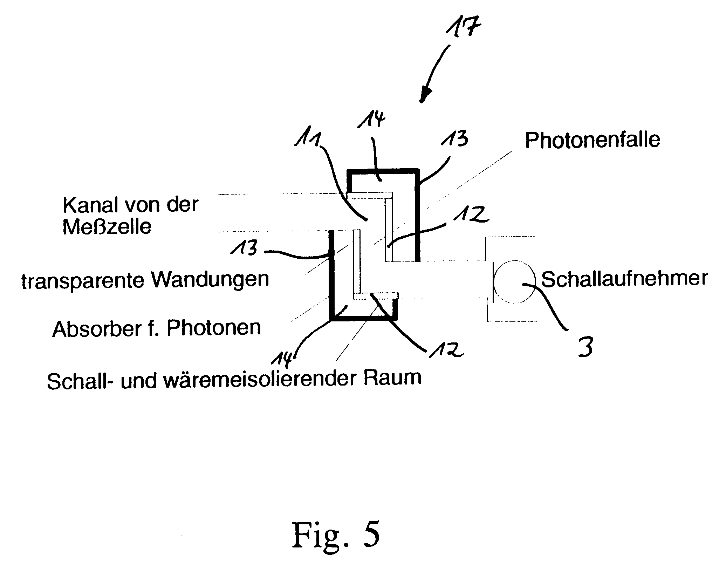

Fig. 5 zeigt eine erfindungsgemäße Ausführungsform einer Photonenfalle, wie sie in Fig. 4 dargestellt ist. Sie kann jedoch ganz allgemein bei jeder geeigneten Anordnung und insbesondere auch bei den anderen erfindungsgemäßen Ausführungsformen zum Einsatz kommen. Die Photonenfalle 17 besteht aus einem für die Strahlung (Photonen) transparenten gekrümmtem Rohr 12, hinter dem sich im Anschluß an einen akustisch und thermisch von dem Kanal 11 isoliertem Raum 14 ein Absorber 13 für Photonen befindet. Die Schallwellen breiten sich in dem mit einem Gas bzw. Luft gefüllten Kanal 11 zum Schallaufnehmer aus, während die Photonen geradlinig die transparente Wand des Rohres 12 durchdringen und von dem Absorber 13 eingefangen werden.

Der erfindungsgemäße Meßkopf kann als kleiner beweglich einsetzbarer Meßkopf oder in einer mehr kompakten Form als sogenanntes "hand-held-Gerät" eingesetzt werden.

Mittels des erfindungsgemäßen Meßkopfes können diskontinuierliche und kontinuierliche sowie qualitative und quantitative Messungen durchgeführt werden.

Eine bevorzugte Verwendung besteht in der nicht-invasiven photoakustischen Bestimmung von Stoffen in biologischen Flüssigkeiten (z.B. Vollblut, Plasma, Serum, Milch, Urin), in Prozeßflüssigkeiten, in Oberflächen-, Trink- und Abwasser, in menschlichen, tierischen, pflanzlichen und bakteriellen Zellverbänden bzw. deren Suspensionen oder Homogenate (z.B. Fermenterbrühen, Zellkulturen), in menschlichen, tierischen und pflanzlichen Geweben und Organen, in Nahrungsmitteln und Arzneimitteln, und im Rahmen von histochemischen, zellbiologischen, immunologischen, molekularbiologischen und klinisch-chemischen Analysenverfahren. Vorteilhafterweise können die Analysenverfahren reagenzienfrei durchgeführt werden.

Ein weiteres Anwendungsgebiet ist die Oberflächenanalytik von Festkörpern.

## Patentansprüche

1. Meßkopf (1) für die photoakustische Spektroskopie mit mindestens einer Strahlungsquelle (2) zur Bestrahlung eines Objekts (4) und einem Schallaufnehmer (3) dadurch gekennzeichnet, daß die mindestens eine Strahlungsquelle (2) in dem Meßkopf (1) angeordnet ist.

2. Meßkopf nach Anspruch 1, gekennzeichnet durch eine einseitig offene Meßkammer (6).

3. Meßkopf nach Anspruch 1 oder 2, dadurch gekennzeichnet, daß mehrere Strahlungsquellen (2) auf einem Substrat (5) im Inneren einer Meßkammer (6) angeordnet sind.

4. Meßkopf nach Anspruch 3, dadurch gekennzeichnet, daß die Strahlungsquellen Leuchtdioden oder Laserdioden sind und als Chips ohne Gehäuse direkt auf dem Substrat (5) angeordnet sind.

5. Meßkopf nach Anspruch 4, dadurch gekennzeichnet, daß das Substrat (5) Teil der Meßkammer (6) ist.

6. Meßkopf nach einem der Ansprüche 1 bis 5, dadurch gekennzeichnet, daß die Strahlung ohne Zwischenschalten optischer Elemente insbesondere Linsen, direkt auf das Objekt (4) gerichtet ist.

7. Meßkopf nach Anspruch 6, dadurch gekennzeichnet, daß die Strahlungsquellen (2) durch einen für die Strahlung transparenten Verguß (18) geschützt sind.

8. Meßkopf nach Anspruch 2, dadurch gekennzeichnet, daß mehrere Strahlungsquellen (2) außerhalb der Meßkammer (6) hinter einem Fenster (7) angeordnet sind.

9. Meßkopf nach Anspruch 8, dadurch gekennzeichnet, daß das Fenster (7) aus für die jeweilige Strahlung transparentem Glas oder Kunststoff besteht.

10. Meßkopf nach einem der Ansprüche 1 bis 9, dadurch gekennzeichnet, daß mehrere Strahlungsquellen (2) außerhalb der Meßkammer (6) in optische Verbindung mit einem Lichtführungselement (8) angeordnet sind, welches für die Strahlung transparent ist und so gestaltet ist, daß die Strahlung nur an gewünschten Orten ein- bzw. austreten kann.

11. Meßkopf nach Anspruch 10, dadurch gekennzeichnet, daß das Lichtführungselement (8) rotationssymmetrisch aufgebaut ist.

12. Meßkopf nach Anspruch 10 oder 11, dadurch gekennzeichnet, daß das Lichtführungselement (8) Teil der Meßkammer (6) ist.

13. Meßkopf nach einem der Ansprüche 10 bis 12, dadurch gekennzeichnet, daß das Lichtführungselement (8) Strahlung von außerhalb der Meßkammer (6) auf das Objekt (4) leitet, wobei die Strahlungsquellen (2) auf einem die Meßkammer (6) umgebenden Substrat (5) angeordnet sind, die Strahlung durch transparente Fenster (15) in das Lichtführungselement (8) eintritt und durch das Auskoppelfenster (16) in die Meßkammer (6) eintritt und auf das Objekt (4) geleitet wird.

14. Meßkopf nach Anspruch 1 oder 2, dadurch gekennzeichnet, daß die Meßkammer (6) ein Hohlkörper (9) ist und mehrere strahlungsquellen (2) außerhalb des Hohlkörpers (9) entlang der Umrandung von dessen Grundfläche so angeordnet sind, daß die Strahlung jeder Strahlungsquelle (2) durch ein Fenster (10) in den Hohlkörper eindringt und über Reflexion oder Streuung an der Innenseite (9a) des Hohlkörpers (9) auf das Objekt (4) geleitet wird.

15. Meßkopf nach Anspruch 14, dadurch gekennzeichnet, daß der Hohlkörper (9) an der Innenseite (9a) verspiegelt ist oder durch eine Beschichtung verlustarm streuend wirkt.

16. Meßkopf nach Anspruch 14 oder 15, dadurch gekennzeichnet, daß der Hohlkörper (9) ein Polyeder oder ein Teil davon, insbesondere eine Pyramide ist.

17. Meßkörper nach Anspruch 14 oder 15, dadurch gekennzeichnet, daß der Hohlkörper (9) ein krummmflächig begrenzter Körper, insbesondere ein Kegel, ein Kugelsegment, ein Paraboloid oder ein Ellipsoid oder jeweils ein Teil davon wie ein Kegelstumpf oder eine Kugelschicht ist.

18. Meßkopf nach einem der Ansprüche 1 bis 17, dadurch gekennzeichnet, daß die Strahlungsquellen Leuchtdioden, Laserdioden, oder eine Kombination von beiden sind.

19. Meßkopf nach einem der Ansprüche 1 bis 18, dadurch gekennzeichnet, daß die Strahlung mittels defraktiver oder refraktiver optischer Elemente auf das Objekt (4) geleitet wird.

20. Meßkopf nach einem der Ansprüche 1 bis 19, dadurch gekennzeichnet, daß die mindestens eine Strahlungsquelle (2) in einem Wellenbereich von 400 nm bis 2500 nm strahlt.

21. Photonenfalle, insbesondere für einen Meßkopf für die photoakustische Spektroskopie nach einem der Ansprüche 1 bis 20, bestehend aus: einem gekrümmten Rohr (11) mit transparenten Wandungen (12), einem schall- und wärmeisolierenden Raum (14) der das Rohr (11) umgibt, und Absorbern für Photonen (13), die im Anschluß an den schall- und wärmeisolierenden Raum (14) angeordnet sind, wobei Schallwellen in dem Rohr (11) transportiert werden und Photonen die transparente Wand (12) des Rohres (11) durchdringen und von den Absorbern (13) absorbiert werden.

22. Verwendung des Meßkopfes nach einem der Ansprüche 1 bis 20 zur Bestimmung von Stoffen in biologischen Flüssigkeiten, insbesondere Vollblut, Plasma, Serum, Milch, Urin; in Prozeßflüssigkeiten; in Oberflächen- Trink- und Abwasser; in menschlichen, tierischen, pflanzlichen und bakteriellen Zellverbänden oder deren Suspensionen oder Homogenate, insbesondere Fermenterbrühen und Zellkulturen; in menschlichen, tierischen und pflanzlichen Geweben und Organen; in Nahrungsmitteln, Arzneimitteln; oder im Rahmen von histochemischen, zellbiologischen, immunologischen, molekularbiologischen und klinisch-chemischen Analysenverfahren.

23. Verwendung nach Anspruch 22, wobei der Gehalt von Glukose im menschlichen Blut bestimmt wird.

24. Verwendung des Meßkopfes nach einem der Ansprüche 1 bis 20 in der Oberflächenanalytik von Festkörpern oder Gasen.
